# EUROPÄISCHE PATENTANMELDUNG

(11) **EP 2 311 413 A1**
(43) Veröffentlichungstag der Anmeldung: **20.04.2011**
(21) Anmeldenummer: 10188110.0
(22) Anmeldetag: 19.10.2010
(51) Int. Cl.: A61F 5/37

(54) **Sicherheitsbandage**

(30) Priorität: 19.10.2009 DE 202009014135 U
(71) Anmelder: Sanchez, Alexander, 21266 Jesteburg (DE)
(72) Erfinder: Sanchez, Alexander, 21266 Jesteburg (DE)
(74) Vertreter: Richter, Werdermann, Gerbaulet & Hofmann

(57) **Zusammenfassung**

Die Erfindung betrifft eine Sicherheitsbandage (100) zur Begrenzung der Beweglichkeit eines auf einem Lager (13) liegenden Patienten (10) bzw. zur Positionierung eines Patienten (10) auf einem Lager (13) mit einem Leibgurt (11), der den Körper des Patienten (10) um den Bauchbereich umschließt, und mit einer Schulterhalterung (16), die entlang der oberen Oberkörperpartie (10a) und über die Schultern des Patienten (10) verläuft, wobei die Schulterhalterung (16) Schultergurte (17, 18) aufweist. Um eine Sicherheitsbandage (100) zur Verfügung zu stellen, bei der insbesondere für Patienten, die sich in Bauchlage befinden, eine schonende Fixierung gewährleistet ist, schlägt die Erfindung vor, dass an der Schulterhalterung (16) mindestens ein Zusatzgurt (22, 23) fest oder lösbar angebracht ist, der sich in Längsrichtung der Sicherheitsbandage (100) erstreckt und am Lager (13) befestigbar ist.

## Beschreibung

Die Erfindung betrifft eine Sicherheitsbandage zur Begrenzung der Beweglichkeit eines auf einem Lager liegenden Patienten bzw. zur Positionierung eines Patienten auf einem Lager mit einem Leibgurt, der den Körper des Patienten um Bauchbereich umschließt, und mit einer Schulterhalterung, die entlang der oberen Oberkörperpartie und über die Schultern des Patienten verläuft, wobei die Schulterhalterung Schultergurte aufweist.

Sicherheitsbandagen sind dem Fachmann in den unterschiedlichsten Ausführungsformen bekannt und geläufig.

Die Sicherheitsbandagen bestehen dabei im Wesentlichen aus einem Haltegurt, der quer über ein Lager oder Bett gelegt und mit Verschlussmitteln fixiert und gespannt wird. Diese Verschlussmittel umfassen in bevorzugter Weise Betthalterungen in Form gurtartiger Bänder, die entweder einstückig mit dem Haltegurt ausgeführt oder fest mit diesem verbunden wie beispielsweise vernäht sind. Endseitig an den Betthalterungen sind zur Befestigung der Betthalterungen an einem seitlichen Rahmenteil des Lagers oder am beweglichen Kopfteil Segelösen ausgebildet. Die Enden der Betthalterungen werden dabei am Bettrahmen und unter Umständen am beweglichen Kopfteil befestigt. Die Befestigung erfolgt mit Magnetknöpfen, die in an den Betthalterungen vorgesehenen Laschen eingesteckt sind. Die Betthalterungen sind derart lang ausgelegt, dass der Haltegurt an beiden Seiten des Lagers befestigt werden kann. Der Haltegurt kann dabei ungefähr die Breite des Lagers aufweisen oder verkürzt ausgebildet sein, wobei hierbei die Betthalterungen entsprechend länger ausgebildet sind.

Des Weiteren umfassen die Sicherheitsbandagen einen Leibgurt, der um den Bauchbereich des Patienten geschlungen und mit geeigneten Verschlussmitteln festgelegt wird. Bei kooperativen Patienten können diese Verschlussmittel ein Klettverschluss oder dergleichen an den Endbereichen des Leibgurtes sein. Bei nicht kooperativen Patienten umfassen die Verschlussmittel vorzugsweise an den Endbereichen des Leibgurts ausgebildete Segelösen, durch die ein an sich bekannter Magnetknopf hindurchsteckbar ist, um die beiden Enden des Leibgurts aneinander zu befestigen. Ein derartiger Magnetknopf kann nur mit einem zusätzlichen Dauermagneten geöffnet werden, so dass dies nicht von einem Patienten selbst vorgenommen werden kann.

Zusätzlich können an dem Leibgurt eine Schulterhalterung in Gestalt von Schultergurten angeordnet sein, um einen Patienten besser fixieren zu können, die im Wesentlichen quer zum Leibgurt ausgerichtet sind und über die Schultern des Patienten geführt werden. Diese Schultergurte sind vorzugsweise an der Rückseite des Leibgurtes oder des Haltegurtes befestigt und werden über die Schultern des Patienten nach vorne geführt und an der Vorder- oder Oberseite des Leibgurtes mit geeigneten Verschlussmitteln befestigt. Derartige Schultergurte gewährleisten, dass der Patient weder bewusst noch unbewusst den Leibgurt über die Hüften nach unten abstreifen und das Lager, beispielsweise ein Bett oder eine speziell vorgesehene Krankenliege, verlassen kann.

Zudem sind die vorbekannten Schulterhalterungen mit einer Verbindung versehen, die die Schultergurte verbinden. Hierbei handelt es sich vornehmlich um sogenannte Steggurte, d. h. Gurte, die bei parallel verlaufenen Schultergurten zwischen den Schultergurten angeordnet sind. Eine weitere vorbekannte Variante sind Schulterhalterungen, die Verbindungen in Gestalt von Kreuzungspunkten, d. h. gekreuzte Schultergurte aufweisen.

Infolge der bei tobenden bzw. aggressiven Patienten notwendigen Fixierung mittels einer Sicherheitsbandage der eingangs genannten Art ergeben sich jedoch zwei Gefahren: Die Verletzung des Pflegepersonals durch den Patienten und eine mögliche Selbstverletzung des Patienten.

Die vorbekannte Sicherheitsbandage ist nur für längerdauernde Fixierung auf dem Rücken liegender Patienten ausgelegt. Die Rückenlage und das damit gegebene freie Sichtfeld ermöglichen dem tobenden bzw. aggressiven Patienten die Wahrnehmung jeder sich bietenden Gelegenheit zur Gewalttätigkeit gegen das Pflegepersonal.

Den Patienten mit der vorbekannten Sicherheitsbandage einfach auf dem Bauch liegend zu fixieren ist insofern problematisch, als sich der Patient mit Heben und Senken des Oberkörpers Gesichtsverletzungen zufügen kann. Zudem ist die vorbekannte Sicherheitsbandage nicht für die Fixierung in Bauchlage ausgelegt. So kann sich der Patient mit den Schultergurten Selbstverletzungen am Gesicht beibringen. Allein das Befestigen der Schultergurte am Schloss des Leibgurtes führt zu einer druckstellenverursachenden Verkantung des Leibgurtes über dem Patienten und erfordert somit eine andersartige Befestigung.

Daher es ist Aufgabe der Erfindung, eine Sicherheitsbandage der eingangs genannten Art zur Verfügung zu stellen, bei der insbesondere für Patienten, die sich in Bauchlage befinden, eine schonende Fixierung gewährleistet ist.

Diese Aufgabe wird mit den Merkmalen des Anspruchs 1 gelöst. Weitere vorteilhafte Ausgestaltungen der Erfindung manifestieren sich in den Unteransprüchen.

Der Kerngedanke der Erfindung besteht darin, dass im Gegensatz zu den vorbekannten Sicherheitsbandagen mindestens ein Zusatzgurt an der Schulterhalterung fest oder lösbar angebracht ist, wobei sich der Zusatzgurt in Längsrichtung der Sicherheitsbandage erstreckt und am Lager befestigbar ist. In einer hier verwendeten Terminologie wird als Längsrichtung der Sicherheitsbandage die Richtung angesehen, die konform mit der Längsrichtung des Lagers, d. h. in Richtung der längsten Ausdehnung des Lagers geht.

Durch die Zusatzgurte wird der Patient am Aufrichten gehindert. Die Zusatzgurte sind darüber hinaus dergestalt an den Schultergurten angebracht, dass sich diese am Kopf des Lagers befinden, damit sich der Patient nicht am Gesicht oder gar Hals verletzen kann. Maßgebliche erfindungsgemäße konstruktive Änderung gegenüber herkömmlichen Sicherheitsbandagen ist also mindestens ein in Längsrichtung der Sicherheitsbandage angeordneter Zusatzgurt d. h. ein in Richtung des Kopfes des Lagers verlaufender Gurt. Hierdurch wird vorteilhafterweise erreicht, dass der Patient sicher in Bauchlage fixiert werden kann.

Der Zusatzgurt ist dabei vorzugsweise mit der Schulterhalterung bzw. mit den Schultergurten fest vernäht oder über mit an der Schulterhalterung bzw. Schultergurten befindlichen Schlaufen, die vorzugweise als Metallschlaufen vorliegen, verbunden.

Vorteilhafterweise sind an dem Leibgurt Schlaufen angebracht, und zwar vorzugsweise an der zur oberen Oberkörperpartie hin verlaufenden Kante des Leibgurtes. Mit diesen Schlaufen wird erreicht, dass Schultergurte über Schlaufen, die vorzugsweise als Metallschlaufen vorliegen, am Leibgurt befestigbar sind, so dass vorteilhafterweise der Leibgurt nicht verkantet und sich die zur unlösbaren Befestigung einer Schulterhalterung notwendigen Schlösser oberhalb des Rückens des Patienten befindet.

Es ist dabei von Vorteil, dass zumindest ein Ende der Schulterhalterung über die Schlaufen mit dem Leibgurt befestigt ist. Zweckmäßigerweise weist die Schulterhalterung zwei Schultergurte auf, die jeweils entlang der oberen Oberkörperpartie sowie über die Schultern des Patienten verlaufen. Hierdurch wird erreicht, dass der Patient in Bauchlage noch stärker am Lager fixiert werden kann. Somit ist ihm weitestgehend das Sichtfeld genommen, so dass der Patient weniger Gelegenheit zu Gewalttätigkeit hat. Dies ist insbesondere dann vor Vorteil, wenn es sich bei dem Patienten um aggressive und gewaltbereite Menschen handelt. Als Vorteil ist auch hervorzuheben, dass durch diese erfindungsgemäße konstruktive Ausgestaltung die Schulterhalterung bzw. die Schultergurte derart angebracht sind, dass sich der Patient mit Ihnen nicht am Gesicht verletzen kann.

Eine praktikable Variante der Erfindung sieht vor, dass die Schultergurte mindestens einen Kreuzungspunkt aufweisen, d. h. gekreuzt verlaufen. Die Schultergurte verlaufen dabei vorzugsweise auf der der Rückenpartie des Patienten zugewandten Seite der Sicherheitsbandage gekreuzt. Alternativ kann auch vorgesehen sein, dass zwischen den Schultergurten ein Steggurt angeordnet und mit den Schultergurten verbunden ist.

Zweckmäßigerweise verlaufen Steggurt und der Kreuzungspunkt im zweiten Drittel der Schulterhalterung. Hierdurch wird vorteilhafterweise erreicht, dass der Patient ohne Strangulationsgefahr in Bauchlage fixiert werden kann. Diese im Rahmen der Erfindung vorgesehene konstruktive Änderung zielt also darauf ab, den Steggurt bzw. Kreuzungspunkt in Richtung des Kopfes des Patienten versetzt anzuordnen.

Eine weitere praktikable Variante der Erfindung sieht vor, dass die Sicherheitsbandage einen Bettgurt aufweist, der aus einem Längsgurt und vorzugsweise mit zum Längsgurt senkrecht angeordneten und mit dem Längsgurt verbundenen Vertikalgurten besteht. Diese Variante der Erfindung hat den Vorteil, dass die Schulterhalterung bzw. die Schultergurte zurückgehalten werden, wobei der Bettgurt wiederum optional mit Befestigungsgurten zur Sicherung gegen Verrutschen ausgestattet werden kann. An dem Bettgurt können auch im Rahmen der Erfindung Zusatzgurte zur zusätzlichen Sicherung der Schulterhalterung bzw. Schultergurte angebracht sein. Vorzugweise sind dabei die Enden der Längsgurte durch Schlaufen der Schultergurte gezogen oder der Bettgurt fest mit den Schultergurten verbunden, insbesondere in Form einer Vernähung.

Eine weitere vorteilhafte Ausgestaltung der Erfindung sieht vor, dass die Ösen der Enden der Schultergurte auf Schlösser des Leibgurtes gesteckt sind.

Als Materialien für die erfindungsgemäße Sicherheitsbandage können sämtliche dem Fachmann bekannte Materialien für Sicherheitsbandagen wie Natur- und Synthetikstoffe verwendet werden. Insbesondere besteht die Sicherheitsbandage aber im Wesentlichen aus einem relativ grob gewebten Nesselstoff, um der Sicherheitsbandage eine ausreichende Festigkeit und Widerstandsfähigkeit zu verleihen.

Die erfindungsgemäße Sicherheitsbandage kann bereits vom Hersteller mit Verbindungen und Zusatzgurten ausgestattet und verkauft werden. In gleicher Weise ist es aber auch möglich, dass von einem Hersteller ein Nachrüstsatz für Sicherheitsbandagen angeboten wird, der vorzugsweise aus einem Leibgurt und einem Haltegurt besteht. Dieser Nachrüstsatz besteht weiterhin im Wesentlichen aus einer Schulterhalterung, die für die Anbringung an den Leibgurt vorgesehen sind. Darüber hinaus besteht der Nachrüstsatz aus einem Zusatzgurt und einem Bettgurt. Dabei kann die Anbringung bzw. Befestigung dieser Teile entweder vom Anwender der Sicherheitsbandage oder von dem Hersteller selbst vorgenommen werden. Beispielsweise kann von dem Anwender eine bereits vorhandene Sicherheitsbandage, bestehend aus einem Leibgurt und einem Haltegurt, an den Hersteller gesandt werden, wobei dieser dann die zusätzlichen Teile, d. h. Schlaufen, Schulterhalterung, Verbindung, Zusatzgurt und Bettgurt an der Sicherheitsbandage anbringt.

Eine praktikable Variante der Erfindung sieht vor, dass der Nachrüstsatz dergestalt ist, dass die Schulterhalterung in Form von Schultergurten wie Zusatzgurten an eine Sicherheitsbandage anbringbar ist. Dies kann dergestalt erfolgen, dass ein Taillengurt der Schulterhalterung durch die hierfür vorgesehen Schlaufen gezogen und über dem Rücken des Patienten mit einem Schloss geschlossen wird. Der Taillengurt kann dabei als Bettgurt ausgeführt sein und an dessen Enden am Bettrahmen mit Schlössern befestigt werden oder die Schultergurte mit dem Leibgurt oder dem Haltegurt der Sicherheitsbandage vernäht werden.

Eine weitere zur Hinderung des Patienten am Aufrichten des Oberkörpers vorteilhafte Ausgestaltung der Erfindung sieht vor, dass sich der Steggurt mindestens bis zum seitlichen Randteil des Lagers erstreckt, wobei der Steggurt an dem seitlichen Rahmenteil des Lagers befestigbar ist.

Zudem betrifft die Erfindung ein Sicherheitssystem mit einem Lager und einer Sicherheitsbandage zur Begrenzung der Beweglichkeit eines auf dem Lager liegenden Patienten bzw. zur Positionierung eines Patienten auf dem Lager mit einem Leibgurt, der den Körper des Patienten um den Bauchbereich umschließt, und mit einer Schulterhalterung, die entlang der oberen Oberkörperpartie und über die Schultern des Patienten verläuft, wobei die Schulterhalterung Schultergurte aufweist. An der Schulterhalterung ist dabei zudem mindestens ein Zusatzgurt fest oder lösbar angebracht und das Lager weist mindestens eine Durchbrechung auf, durch die der Zusatzgurt geführt ist und an dem Lager befestigbar ist, um den Patienten am Aufrichten des Oberkörpers zu hindern.

Nachstehend wird die Erfindung anhand von Zeichnungen näher erläutert. Es zeigen in schematischer Darstellung:
- Fig. 1: in Draufsicht eine Sicherheitsbandage gemäß der Erfindung;
- Fig. 2: die Sicherheitsbandage aus Fig. 1 in einer seitlichen Schnittansicht; und
- Fig. 3: einen Bettgurt gemäß der Erfindung.

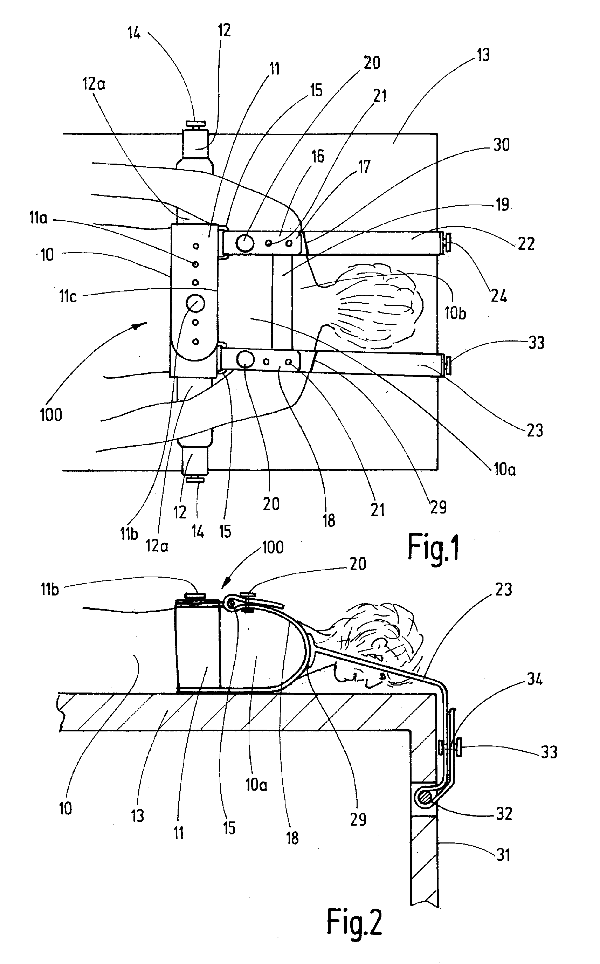

Fig. 1 zeigt eine Sicherheitsbandage gemäß der Erfindung, die mit dem Bezugszeichen 100 versehen ist.

Die Sicherheitsbandage 100 weist einen Haltegurt 12a und einen auf dem Haltegurt 12a befindlichen Leibgurt 11 auf. Der Haltegurt 12a ist an seinen Enden mit Betthalterungen 12 in Form gurtartiger Bänder versehen, um den Haltegurt 12a quer über das Lager 13 auf dieses aufzulegen und mit den Betthalterungen 12 bspw. an einem seitlichen Rahmenteil des Lagers 13 festzulegen und zu verspannen. Hierzu sind die Betthalterungen 12 in an sich bekannter Weise mit in Taschen angeordneten Magnetschlössern 14 sowie mit zugehörigen Segelösen versehen. Der Leibgurt 11 verfügt über Verschlussmittel in Form von Segelösen 11 a und Magnetschlössern 11 b, um ihn um den Bauchbereich des in Bauchlage auf dem Lager liegenden Patienten 10 zu schlingen und die Enden des Leibgurts 11 aneinander festzulegen.

Der Leibgurt 11 ist mit Schlaufen 15 versehen und zwar derart, dass die Schlaufen 15, die vorzugsweise in Form von Metallschlaufen vorliegen, an der zur oberen Oberkörperpartie 10a hin verlaufenden Kante 11 c des Leibgurtes 11 angebracht sind. Die Schlaufen 15 befinden sich dabei auf der den Betthalterungen 12 abgewandten Seite des Leibgurts 11. Die Anbringung der Schlaufen 15 kann dabei vorzugsweise derart erfolgen, dass an der Kante 11 c des Leibgurts 11 sich eine Schlinge befindet, die über eine Naht mit dem Leibgurt 11 vernäht ist. Durch die Schlinge sind die in sich geschlossenen Schlaufen 15, die vornehmlich aus Metall sind, geführt. Die in Fig. 1 gezeigte Sicherheitsbandage 100 verfügt weiterhin über eine Schulterhalterung 16 mit zwei Schultergurten 17,18, die im Wesentlichen parallel zueinander und entlang der oberen Oberkörperpartie 10a des Patienten 10 verlaufen und über die Schultern des Patienten 10 geführt sind. Die Schultergurte 17, 18 sind durch die Schlaufen 15 gezogen, umgeschlagen und deren Ösen 21 auf die Schlösser 20 gesteckt, so dass die Schulterhalterung 16 unlösbar für den Patienten 10 geschlossen ist.

Zwischen den Schultergurten 17, 18 ist ein Steggurt 19 angeordnet, der parallel zum Halte- und Leibgurt 11, 12a und in der Schulterblattpartie 10b des Patienten 10 verläuft sowie die Schultergurte 17, 18 zusammenhält. Der Steggurt ist mit den Schultergurten 17, 18 fest vernäht und liegt auf dem Rücken des Patienten 10 an, d. h. der Steggurt verläuft oberhalb des Körpers des auf dem Lager liegenden Patienten 10 und ist vorzugsweise im zweiten Drittel der Schulterhalterung 16 bzw. der Schultergurte 17, 18 angeordnet. Der Steggurt muss, sobald der Patient 10 auf dem Lager 13 liegt, mit den Schultergurten 17, 18 über den Kopf des Patienten 10 gestülpt werden, damit die Enden der Schultergurte 17, 18 durch die Schlaufen 15 gezogen werden können.

Erfindungswesentliches Merkmal der erfindungsgemäßen Sicherheitsbandage 100 sind Zusatzgurte 22, 23 , die beispielsweise über eine Naht 29, 30 mit den Schultergurten 17, 18 fest verbunden sind und mittels Magnetschlössern 24 mit dem Lager 13 verbunden sind.

Die Zusatzgurte 22, 23 verlaufen im Wesentlichen parallel zueinander und erstrecken sich, wie aus Fig. 2 hervorgeht, von den Schultern des Patienten 10 in Längsrichtung zu der Sicherheitsbandage 100 bis zu den Lagerbeinen 31. Die Längsrichtung der Sicherheitsbandage 100 geht konform mit der Längsrichtung des Lagers 13. An den Lagerbeinen 31 befinden sich Holme 32, um die die Zusatzgurte 22, 23 geführt sind. Die Zusatzgurte 22, 23 weisen Ösen 34 auf, auf die die Magnetschlösser 33 gesteckt sind, so dass die Zusatzgurte 22, 23 für den Patienten geschlossen ist. An der Naht 29, 30 können anstelle des fest vernähten Zusatzgurtes 22, 23 auch Schlaufen, vorzugsweise Metallschlaufen angebracht sein, damit analog zur Verbindung von Leib- und Schultergurten 11, 22, 23 eine lösbare Anbringung des Zusatzgurtes 22, 23 an den Schultergurten 17,18 gegeben ist.

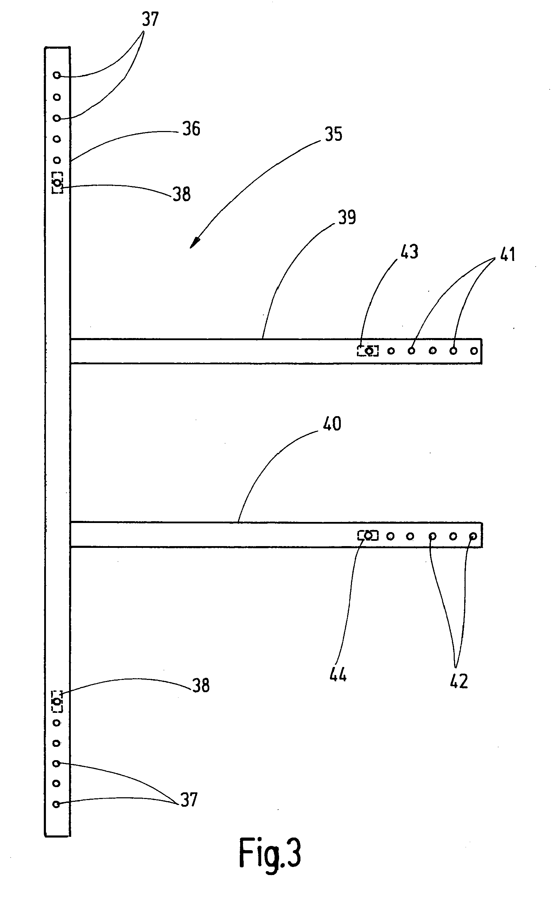

Die in den Fig. 1 und 2 gezeigte Sicherheitsbandage 100 kann zudem einen in Fig. 3 dargestellten Bettgurt 35 aufweisen, der aus einem mit Ösen 37 und Magnetschlössern 38 versehenen Längsgurt 36 und mit zum Längsgurt 36 senkrecht angeordneten und mit dem Längsgurt 36 verbundenen Vertikalgurten 39, 40 besteht. Die Vertikalgurte 39, 40 sind parallel zueinander angeordnet und sind wie der Längsgurt 36 sowohl mit Ösen 41, 42 als auch mit Magnetschlössern 43, 44 versehen.

Der in Fig. 3 gezeigte Bettgurt 35 dient als Alternative, die in den Fig. 1 und 2 gezeigten Schultergurte 17, 18 am Lager 13 zurückzuhalten. Dies geschieht dergestalt, dass der Bettgurt 35 durch in Fig. 3 nicht dargestellte, im Brustbereich des in Fig. 1 und Fig. 2 gezeigten Patienten 10, also zwischen Lager 13 und Patienten 10 befindliche Schlaufen, die an den Schultergurten 17, 18 angebracht sind, gezogen oder direkt, also ohne Schlaufen, über die Schultergurte 17, 18 gespannt und anschließend dessen Ende am Gestell des Lagers 13 mit Schlössern befestigt werden, wobei die Vertikalgurte 39, 40 in herkömmlicher Weise an den Lagerbeinen bzw. Rahmenteilen des Lagers 13 befestigt werden. Im Rahmen der Erfindung ist es zudem möglich, dass an den Vertikalgurten 39, 40 Zusatzgurte zur zusätzlichen Sicherung der Schultergurte 17, 18 angebracht sind. Hierzu können Metallschlaufen an den Schultergurten 17, 18 vorgesehen sein, um die Vertikalgurte 39, 40 mit den Zusatzgurten zu verbinden.

Bezugzeichenliste
- 100: Sicherheitsbandage
- 10: Patient
- 10a: Oberkörperpartie
- 10b: Schulterblattpartie
- 11: Leibgurt
- 11a: Segelösen
- 11b: Magnetschloss
- 11c: Kante
- 12: Betthalterung
- 12a: Haltegurt
- 13: Lager
- 14: Magnetschloss
- 15: Schlaufe
- 16: Schulterhalterung
- 17: Schultergurt
- 18: Schultergurt
- 19: Steggurt
- 20: Schloss
- 21: Öse
- 22: Zusatzgurt
- 23: Zusatzgurt
- 24: Magnetschloss
- 29: Naht
- 30: Naht
- 31: Lagerbein
- 32: Holm
- 33: Magnetschloss
- 34: Öse
- 35: Bettgurt
- 36: Längsgurt
- 37: Öse
- 38: Magnetschloss
- 39: Vertikalgurt
- 40: Vertikalgurt
- 41: Öse
- 42: Öse
- 43: Magnetschloss
- 44: Magnetschloss

## Patentansprüche

1. Sicherheitsbandage (100) zur Begrenzung der Beweglichkeit eines auf einem Lager (13) liegenden Patienten (10) bzw. zur Positionierung eines Patienten (10) auf einem Lager (13) mit einem Leibgurt (11), der den Körper des Patienten (10) um den Bauchbereich umschließt, und mit einer Schulterhalterung (16), die entlang der oberen Oberkörperpartie (10a) und über die Schultern des Patienten (10) verläuft, wobei die Schulterhalterung (16) Schultergurte (17, 18) aufweist, **dadurch gekennzeichnet, dass** an der Schulterhalterung (16) mindestens ein Zusatzgurt (22, 23) fest oder lösbar angebracht ist, der sich in Längsrichtung der Sicherheitsbandage (100) erstreckt und am Lager (13) befestigbar ist.

2. Sicherheitsbandage nach Anspruch 1, **dadurch gekennzeichnet, dass** an dem Leibgurt (11) Schlaufen (15) angebracht sind.

3. Sicherheitsbandage nach Anspruch 2, **dadurch gekennzeichnet, dass** die Schlaufen (15) an der zur oberen Oberkörperpartie (10a) hin verlaufenden Kante (11 c) des Leibgurtes (11) angebracht sind.

4. Sicherheitsbandage nach einem der Ansprüche 1 bis 3, **dadurch gekennzeichnet, dass** die Schlaufen (15) Metallschlaufen sind.

5. Sicherheitsbandage nach einem der Ansprüche 1 bis 4, **dadurch gekennzeichnet, dass** zumindest ein Ende der Schulterhalterung (16) über die Schlaufen (15) mit dem Leibgurt (11) befestigt ist und/oder die Schulterhalterung (16) zwei Schultergurte (17, 18) aufweist und/oder zwischen den Schultergurten (17, 18) ein Steggurt (19) angeordnet und mit den Schultergurten (17, 18) verbunden ist.

6. Sicherheitsbandage nach einem der Ansprüche 1 bis 5, **dadurch gekennzeichnet, dass** die Schultergurte (17, 18) mindestens einen Kreuzungspunkt aufweisen.

7. Sicherheitsbandage nach Anspruch 5 oder 6, **dadurch gekennzeichnet, dass** der Steggurt (19) und der Kreuzungspunkt im zweiten Drittel der Schulterhalterung (16) verlaufen und/oder sich der Steggurt (19) mindestens bis zum seitlichen Rahmenteils des Lagers erstreckt, wobei der Steggurt (19) an dem seitlichen Rahmenteil des Lagers (13) befestigbar ist.

8. Sicherheitsbandage nach einem der vorhergehenden Ansprüche, **dadurch gekennzeichnet, dass** der Zusatzgurt (22, 23) im Schulterbereich des Patienten (10) an der Schulterhalterung (16) angebracht ist.

9. Sicherheitsbandage nach einem der Ansprüche 1 bis 8, **dadurch gekennzeichnet, dass** die Bandage (100) einen Bettgurt (35) aufweist, der aus einem Längsgurt (36) besteht.

10. Sicherheitsbandage nach Anspruch 9, **dadurch gekennzeichnet, dass** der Bettgurt einen zum Längsgurt (36) senkrecht angeordneten und mit dem Längsgurt (36) verbundenen Vertikalgurten (39, 40) aufweist und/oder die Enden des Längsgurts (36) durch Schlaufen der Schultergurte (17, 18) gezogen sind oder der Bettgurt fest mit den Schultergurten (17, 18) verbunden ist, insbesondere in Form einer Vernähung.

11. Sicherheitsbandage nach einem der vorhergehenden Ansprüche, **dadurch gekennzeichnet, dass** Leibgurt (11), Schulterhalterung (16), Zusatzgurt (22, 23) und Bettgurt (35) mit Ösen (11a, 21, 34, 37, 41, 42) versehen sind und/oder Leibgurt (11), Schulterhalterung (16), Zusatzgurt (22, 23) und Bettgurt (35) mit Magnetschlössern (11 b, 14, 24, 33, 38, 43, 44) versehen sind und/oder die Sicherheitsbandage (100) einen Haltegurt (12a) aufweist und/oder ein Ende der Schulterhalterung (16) mit dem Haltegurt (12a) oder dem Leibgurt (11) vernäht ist und/oder der Haltegurt (12a) und/oder der Zusatzgurt (22, 23) und/oder der Bettgurt (35) am Lager (13) befestigbar ist.

12. Sicherheitsbandage nach Anspruch 1, **dadurch gekennzeichnet, dass** Ösen (21) der Enden der Schultergurte (17, 18) auf Schlösser des Leibgurts (11) gesteckt sind.

13. Nachrüstsatz für eine Sicherheitsbandage (100) zur Begrenzung der Beweglichkeit eines auf einem Lager (13) liegenden Patienten (10) bzw. zur **Positionierung** eines Patienten (10) auf einem Lager (13) mit einem Leibgurt, (11) der den Körper des Patienten (10) um den Bauchbereich umschließt, und mit einer Schulterhalterung (16), die entlang der oberen Oberkörperpartie (10a) und über die Schultern des Patienten (10) verläuft umfassend Schultergurte (17, 18) in der Schulterblattpartie (10b) oder unmittelbar unterhalb der Schulterblattpartie (10b) des Patienten (10) und Zusatzgurte (22, 23).

14. Nachrüstsatz nach Anspruch 13, **dadurch gekennzeichnet, dass** die Schulterhalterung (16) in Form von Schultergurten (17, 18) mit den Zusatzgurten (22, 23) an eine Sicherheitsbandage (100) anbringbar sind.

15. Sicherheitsbandage (100) zur Begrenzung der Beweglichkeit eines auf einem Lager (13) liegenden Patienten (10) bzw. zur Positionierung eines Patienten (10) auf einem Lager (13) mit einem Leibgurt (11), der den Körper des Patienten (10) um den Bauchbereich umschließt, und mit einer Schulterhalterung (16), die entlang der oberen Oberkörperpartie (10a) und über die Schultern des Patienten (10) verläuft, **dadurch gekennzeichnet, dass** an der Schulterhalterung (16) mindestens ein Zusatzgurt (22, 23) fest oder lösbar angebracht ist, der sich von den Schultern des Patienten (10) weg erstreckt und am Lager (13) befestigbar ist.

16. Sicherheitssystem mit einer Sicherheitsbandage (100) und einem Lager (13) zur Begrenzung der Beweglichkeit eines auf dem Lager (13) liegenden Patienten (10) bzw. zur Positionierung eines Patienten (10) auf dem Lager (13) mit einem Leibgurt (11), der den Körper des Patienten (10) um den Bauchbereich umschließt, und mit einer Schulterhalterung (16), die entlang der oberen Oberkörperpartie (10a) und über die Schultern des Patienten (10) verläuft, wobei die Schulterhalterung (16) Schultergurte (17, 18) aufweist, **dadurch gekennzeichnet, dass** an einer Schulterhalterung (16) mindestens ein Zusatzgurt (22, 23) fest oder lösbar angebracht ist und das Lager (13) mindestens eine Durchbrechung aufweist, wobei der Zusatzgurt (22, 23) durch die Durchbrechung geführt ist und an dem Lager (13) befestigbar ist.
